# EUROPEAN PATENT APPLICATION

(11) **EP 0 803 208 A1**
(43) Date of publication of application: **29.10.1997**
(21) Application number: 96302840.2
(22) Date of filing: 23.04.1996
(51) Int. Cl.: A43B 17/03, A43B 17/14

(54) **Insole**

(71) Applicant: Japan Health Supply, Inc., Tokyo (JP)
(72) Inventor: Nakagawa, Masahiko, Tokyo (JP)
(74) Representative: Rees, David Christopher

(57) **Abstract**

An insole which contributes to correct flatfoot and promote health is to be provided. The insole 1 of this invention comprises a tightly sealed bladder formed of two flexible resin sheets 2(3) having the edged parts 4 thereof fused and a liquid 5 filled in the bladder to such an extent as that the two sheets 2(3) partially come into mutual contact when a certain load is exerted thereon, characterized in that a protruded part 11 is formed from the shoe sole-shaped tightly sealed bladder. For flat-footed persons, the insole can easily correct flatfoot by forcibly forming a developed arch on the foot sole thereof. For normal persons, the insole can stimulate the entire arch, apply a sufficient pressing force equivalent to that by stepping (on a green bamboo) to foot soles, and manifest a satisfactory massaging effects.

## Description

### BACKGROUND OF THE INVENTION

### Field of the Invention:

This invention relates to a novel insole. More particularly, this invention relates to an insole which manifests a profound effect in the correction of flatfoot and, even for a person having normal arches, stimulates the whole arch and promotes his health.

### Description of the Prior Art:

From the medical point of view, human bodies by nature are not so built as to keep standing for a long time. Human beings, therefore, find it more painful to keep standing than to walk or sit. The joints of the bones, particularly those of the knees, rely on the cartilage as cushioning materials for balancing himself on his legs in the process of standing, walking, etc. A flat-footed person who has flat soles showing virtually no arches, whether congenital or acquired, experiences difficulty in balancing himself on his legs and consequently tends to develop the symptom of being readily fatigued as by walking or standing continuously as compared with a healthy person who has normal arches of the feet. Particularly when the person is compelled to keep standing for a long time on a hard floor as of concrete or marble, he ultimately complains of swollen feet and lumbago because of degradation of systemic circulation of blood and overload on his legs and waist. These symptoms have been growing in flat-footed persons, particularly those who have acquired flatfeet due to continuous standing for a long time, and can be called as modern diseases. The same diseases are occurred for aged persons whose cartilage as of knee joints as cushioning material have been worn with the elapse of time to such a degree as that they no longer manifest a cushioning effect.

For the surgical, particularly orthopedic, correction of the flatfeet which may well be called as a modern disease, a method which consists in artificially and forcibly forming an arch on a sole of a flat-footed person by placing on the part of the shoe sole corresponding to his flatfoot a flatfoot-correcting device made of such a flexible material as polyurethane or silicone resin in such a shape as comprising an upper swelled part conforming in contour to the flatfoot and a lower flat lunar part (as disclosed in JP-U-02-111,419, for example) is available. When this correcting device is made of such a flexible material as polyurethane, it is incapable of producing a satisfactory massaging effect because it is virtually destitute of the pressing force obtainable by the exercise of stepping on a green bamboo which is claimed to be good for health. When the correcting device is made of such a relatively elastic flexible material as silicone resin, it entails the disadvantage that the projection of the swelled part thereof constantly exerts pressure on the flat-footed area, causes the user to be rather fatigued than heeled after using the device for a period exceeding a certain length, and compels him to give up the further use of the device. Further, since the flatfoot-correcting device of this sort is generally marketed as fixed in shape and size, it cannot fully conform to the contour of the arch of an individual user.

It has been known to date that the foot soles have an intimate bearing on the health of various areas of the human body. The zone therapy has found recognition as a method for treating disorders of various internal organs by pressing and stimulating various zones of the foot soles ("effective spots" and so-called "tsubo" in Japanese) which have been empirically associated with the conditions of internal organs. As one example of the zone therapy, the exercise of stepping (such as, for example, on a green bamboo) for the exertion of pressure on various areas (effective spots) of the soles may be used. This exercise serves the purpose of not merely inciting physical motions only of the feet but also moving the muscles of the entire system, promoting the circulation of blood, and recovering from the fatigue as demonstrated from the test results with an electromyography. Since the effective spots communicating with the internal organs of the patient's body are concentrated on foot soles, this exercise further results in stimulating the effective spots and exalting the functions of the internal organs. It is safely concluded that the stimulation exerted on the soles as by the stepping has the dual effects as mentioned above and contributes to promoting the health of various areas (internal organs) of the patient's body associated with the effective spots on the soles. Appropriately, therefore, pressure ought to be exerted moderately on as many effective spots as permissible.

As a means for massaging the entire soles of a patient so as to ensure manifestation of the pressing effect mentioned above, insoles manufactured by fusing matched edges of two sheets of an ethylene-vinyl acetate copolymer thereby forming a tightly sealed bladder, exposing the tightly sealed bladder to radiation, and filling the sealed bladder with a liquid to such an extent as that the two sheets of the bladder may partially come into mutual contact when a load is exerted on the interior of the tightly sealed bladder have been proposed in JP-A-60-72,501, JP-A-60-72,502, JP-A-01-126,905, JP-A-01-126,906, and JP-A-06-133,805, for example.

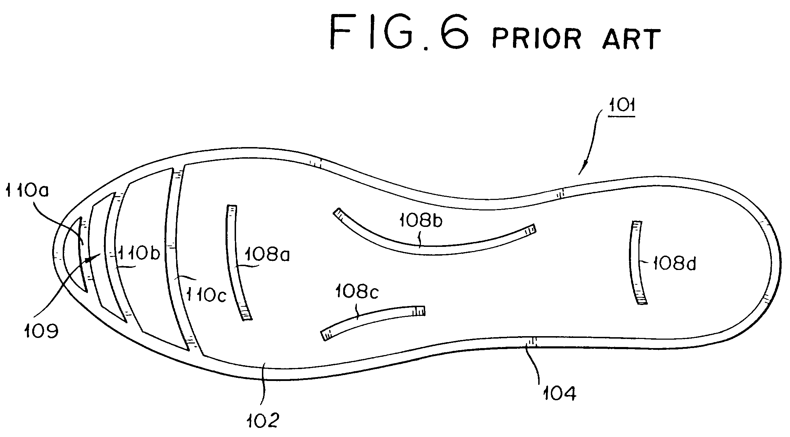

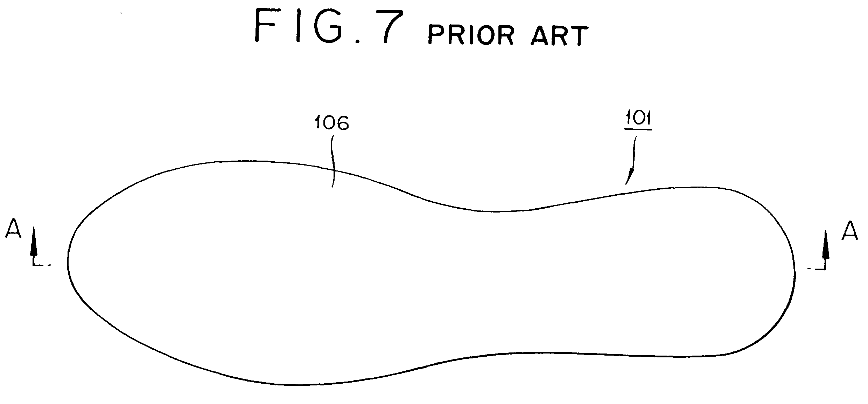

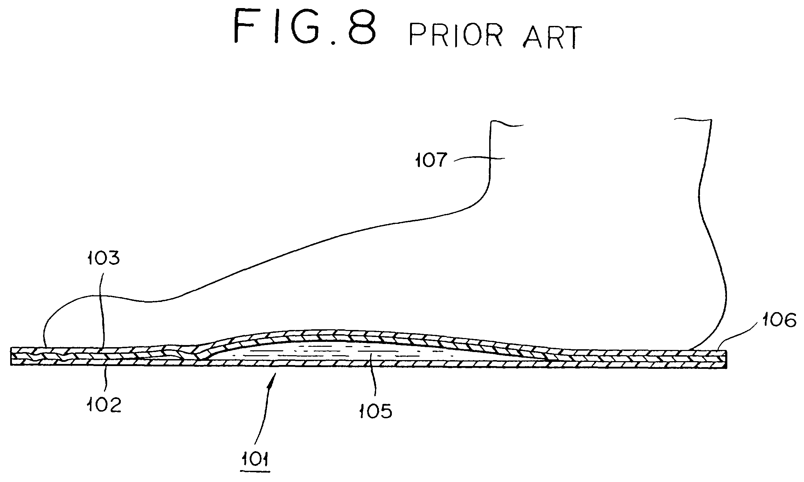

Typical examples of the insoles which are disclosed in the patent publications mentioned above are shown in Figs. 6 to 8. Fig. 6 is a schematic plan view of a concrete example of the left insole produced by a conventional method. Fig. 7 is a bottom view of Fig. 6. Fig. 8 is a cross section taken through Fig. 6 along the line A-A, showing the insole of Fig. 6 placed in a shoe and actually put to use.

As shown in Figs. 6 to 8, an insole 101 manufactured by a conventional method comprises a tightly sealed bladder resulting from fusing matched edges 104 of two superposed sheets 102, 103 each in the shape of a foot sole, made of an ethylene-vinyl acetate copolymer, and partially filled with propylene glycol as a liquid 105 in the inner space thereof. The portion of the insole on which the load is exerted by a foot 107 of a user varies, as shown in Fig. 7, with the equilibrium during the use thereof. The amount of the liquid 105 to be filled in the bladder is such that the two sheets 102, 103 may come into mutual contact in the part on which the exerted load is heavy and the liquid 105 may move to the part on which the exerted load is light. In the central part of the tightly sealed bladder, barriers 108a to 108d against the liquid 105 are further formed as partially fused to the two superposed sheets 102, 103. In toe tip parts 109 of the matched edges 104 of the two sheets 102, 103, at least one fused part, for example, fused parts 110a to 110c as shown in Fig. 6, is formed as connected to the lateral side of the matched edges 104 and the inner space of the tightly sealed bladder before the innermost fused part 110c is used as a cushioning material. The insole 101 with this structure, therefore, can be cut along the fused part 110a or 110b, to fit for the size of a shoe to be used.

The ethylene-vinyl acetate copolymer of which the sheets 102, 103 mentioned above are formed is a copolymer consisting of ethylene and vinyl acetate and having a vinyl acetate content in the range of 5 to 30% by weight. The copolymer has a number average molecular weight in the range of 10,000 to 150,000. The thickness of the sheets is in the range of 0.3 to 1.0 mm. The dosage of the radiation used on the sheets is in the range of 4 to 10 Mrad at 20 °C in an ambience of nitrogen gas. The source of this radiation is electron rays or γ ray. A Co-60 gamma ray may be used as the source of γ ray and a resonance transformer as the electron beam source.

A cushioning layer 106 may be used on the foot sole side of the tightly sealed bladder as shown in Fig. 7. For the cushioning layer 106, a woven fabric, a non-woven fabric, a raised fabric, a knitted fabric, a vinyl leather, a natural leather, or a synthetic leather may be used. When the cushioning layer 106 is elected to be used at all, it may be substantially identical in shape with the insole 101 which is ultimately obtained.

The insoles manufactured by conventional methods can be invariably placed in intimate contact with the shoe soles. They are made to conform veritably to the shoe soles lest they should loosely move or change places inside the shoes. They are shaped so as to meet perfectly the purpose of exerting pressure on the entire soles of users' feet. While the users are in the process of walking, therefore, their loads are exerted on either the heels or the bases of the toes and the liquid filled in the insole moves to a portion having no load exerted thereon. The insoles, therefore, are enabled to exert pressure evenly on the entire soles of the users' feet in the long run. The users are merely required to stand and walk for the purpose of having the entire soles of their feet pressed appropriately by the insoles.

In the insoles which are so made as to conform in shape to the shoe soles, the load exerted to the heels and the bases of the toes while the users keep standing compels the liquid filled in the insoles to push up the parts of the soles of the users' feet which are not exposed to the load (chiefly the parts of the shoe soles corresponding to the arch of the users' foot). In this case, the insoles are capable of stimulating only a part of the arch confronting the shoe bottom. The pressing force which can stimulate the arch part of the user's foot during standing, therefore, is naturally limited by the shape of a shoe bottom. These insoles, accordingly, are incapable of exerting ample pressing force on the entire arch which has a three-dimensional structure and complicated curved faces and further has a different depressed contour depending on each user. The insoles conforming in the shape to a shoe sole decrease the empty spaces inside the users' shoes and make the shoes too tight to abhor continued use of the shoes. Particularly when these insoles are used in high-heeled shoes or boots, they are at a disadvantage in causing pains to the insteps of the users' feet after put for a long time. Even the insoles 101 conforming in the shape of a shoe sole which can be cut along the fused part 110a or 110b depending on the sizes of the shoes selected can not conform to all the shoe sizes, and must be manufactured in suitable size groups. In the case of free-size insoles designed to conform to all shoe sizes, the conformity aimed at cannot be easily attained solely by cutting these insoles along the fused parts only in the toe tip parts. These insoles, therefore, require to be provided with many fused parts enough to allow insertion of a cut throughout the entire edge part of an insole. They are at a disadvantage in decreasing the inner spaces capable of filling therein the liquid which substantially produces the pressing force to be required. Moreover, since they have many fused parts for cutting, they entail the possibility that the users, while cutting along the fused part, will inflict a cut on the part containing the liquid to cause leakage of the liquid.

Such insoles as are limited only to the toe tip parts (produced under trademark designation of "Half Insole") or to the heel parts (produced under trademark designation of "Heel Cushion") of high-heeled shoes for female users utilize such phenomenon that a load is exerted mainly on the toe tip parts and the heel parts while the users are in the process of standing or walking. These insoles which are made of polyurethane for the purpose of producing a cushioning effect against the impact of a foot and manifesting a massaging effect have been already marketed.

The insoles of this class are chiefly intended for the parts destined to be exposed to the load. The insoles which are intended for the peripheral parts (central parts) including arches, i.e. the parts exposed to a rather small of load, are thought to incur difficulty in manifesting the effect mentioned above. The insoles which are limited only to the peripheral parts including arches have not yet been marketed except for those such as a flatfoot-correcting device mentioned above. No inventions have yet been disclosed concerning an insole which is limited only to the peripheral part including an arch.

It is widely known that most effective spots on the foot sole are concentrated on arches of feet, that the massage given to the arch produces an outstanding effect of healing fatigue as compared with the other parts of the foot sole, and that the exercise of "stepping on a green bamboo" which presses and stimulates arches can be performed at ordinary homes. The insoles which are particularly provided with projections in the portions corresponding to those stepped by arches have been already invented (as disclosed in JP-U-03-60,506 and JP-U-05-11,805, for example).

These insoles, like the flatfoot-correcting device mentioned above, have the disadvantage that their projections constantly exert pressure solely on the same parts of the arch and compel the users to suffer growing fatigue to such an extent as that they abhor continued use of the insoles.

This invention, therefore, aims to solve such problems of the prior art as mentioned above. Specifically, it is an object of the present invention to provide a novel insole which functions excellently in correcting flatfoot and promoting health.

Another object of this invention is to provide an insole which easily corrects flatfoot by imparting a state of fully developed arch forcibly to the flatfoot, exerts stimulation to the entire area of the arch for healthy persons free from flatfoot, suitably generates ample pressing force equivalent to that produced by the exercise of stepping (on a green bamboo) and consequently manifests a massaging effect sufficiently and, therefore, functions excellently in correcting flatfoot and promoting health.

A further object of this invention is to provide an insole which, by utilizing a load exerted by a user's foot sole on a toe tip part, a heel part, or a peripheral part thereof including an arch part, automatically controls the shape of its own so as to manifest pressing force for adjusting in conformity to the contour of the user's own arch, and produces an outstanding effect in correcting flatfoot and promoting health.

A further object of this invention is to provide an insole which avoids making the user's foot in the shoe tight or causing a pain to the foot even after a protracted use and produces an excellent effect in correcting flatfoot or promoting health.

A further object of this invention is to provide an insole which permits the size thereof to be freely selected and produces an excellent effect in correcting flatfoot or promoting health.

A further object of this invention is to provide an insole which excels in weatherability and durability, imparts ample pressing force to the entire sole of the user's foot, and produces an excellent effect in correcting flatfoot or promoting health.

A further object of this invention is to provide an insole which eliminates or alleviates the fatigue caused in the legs and the waist by the toil of the user's feet resulting from continued standing, gives a comfortable touch to the sole of the user's foot, exhibits high hygroscopicity, very rarely moistens the foot with fume, and produces an excellent effect in correcting flatfoot or promoting health.

A further object of this invention is to provide an insole which alleviates the impact to be generated on a heel part when shoes, particularly high-heeled shoes are put on.

### SUMMARY OF THE INVENTION

In order to achieve the above-mentioned objects, the present inventors had carried out relevant research on a novel insole which contribute to correct flatfoot and to promote health, to find that by forming a protruded part in (a) a conventional insole in the shape of a shoe sole which comprises a tightly sealed bladder formed of two flexible resin sheets by fusing the edged parts thereof and a liquid filled in the bladder to such an extent as that the two sheets partially come into mutual contact when a load is exerted thereon or (b) an insole with non-conventional shape which is formed only from a central part of the conventional insole (in this case, the term "central part" represents a part corresponding to an arch part provided that a shoe sole is divided into three parts, i.e. a toe tip part, an arch part and a heel part) so as to obtain such an effect as of forming an arch on a foot sole and of stimulating an entire arch of a normal person, and placing the insole inside a shoe so that the protruded part press a whole area of an arch, namely on a portion between a sole and a lateral side of a foot, in particular with folded in contour to the lateral side of a shoe, most of the liquid filled in the tightly sealed bladder is transferred to or concentrated on the protruded part when a load is exerted on a toe tip part and a heel part or a surrounding part of an arch. They also found that by this action, since a pressing force can be exerted on the whole part corresponding to the arch and an arch can be formed on a foot sole of a flat-footed person to a normal person, flatfoot can be easily corrected without any pains and fatigues. Further, for healthy persons having arches, the protruded part can be excellently fit for a shape of an arch by transferring the liquid filled in the tightly sealed bladder thereto and changing its shape flexibly independent on the size and shape of the arch and a suitable pressing force which is equivalent to that by stepping (on a green bamboo) can be imparted to the arch. At the same time, since the liquid filled in the insole is transferred to portions other than the protruded part during the movement of foot or walking, the pressing force is not continuously exerted only on an arch and therefore the insole of this invention can be used over a long period. By utilizing an insole comprising only a central part which corresponds to an arch part of the shoe-shaped insole, such a defect as of making the inside space (particularly, size in the high-backed direction) of the shoe too tight to make the shoes put in with much difficulties and to hurt the user's feet can be overcome. When an insole is limited only to the central part of the insole, since the differences in width of soles by its size is smaller than those in length and the widthwise slip of the insole can be prevented by providing an insole-fixing material on the surface of the insole contacting with a shoe sole, this type of insole can be used any size of shoes. Further, by selecting a polyhydric alcohol as a liquid, the amount of the liquid filled is not decreased even after the long conservation, excellent weatherability and durability can be obtained with the freezing thereof prevented in actual use, a larger pressing force can be suitable imparted to a whole foot sole. Further, by additionally providing a cushioning layer on the surface of the tightly sealed bladder contacting with a foot sole, the fatigue of feet and waist by continuous standing can be released or alleviated, excellent feeling can be imparted to foot soles, and the improvement of absorbency to make foot soles less humid can be obtained. On the basis of this knowledge, the present invention has been accomplished.

In summary, the objects of this invention can be accomplished (1) an insole which comprises a tightly sealed bladder formed of two flexible resin sheets having the edged parts thereof fused and a liquid filled in the bladder to such an extent as that the two sheets partially come into mutual contact when a certain load is exerted thereon, characterized in that a protruded part is formed from the shoe sole-shaped tightly sealed bladder.

The objects can be also accomplished by (2) an insole set forth in the (1) above, wherein the protruded part is formed for the purpose of correcting a flatfoot by the pressing force of the filled liquid to be generated when a load is exerted on a toe tip part, a heel part or a surrounding part of the arch and to produce an arch on the flatfoot.

The objects can be also accomplished by (3) an insole set forth in the (1) above, wherein the protruded part is formed for the purpose of stimulating an whole area of an arch with the pressing force to be generated when a load is exerted on a toe tip part, a heel part or a surrounding part of the arch and to be equivalent to that produced by the exercise of stepping, and consequently can promote health.

The objects can be further accomplished by (4) an insole set forth in any of the (1) to (3) above, wherein the liquid is at least one member selected from the group consisting of water and polyhydric alcohols.

The objects can be further accomplished by (5) an insole set forth in the (4) above, wherein the liquid is either propylene glycol or glycerin.

The objects can be further accomplished by (6) an insole set forth in the (4) above, wherein the liquid is an aqueous polyhydric alcohol solution.

The objects can be further accomplished by (7) an insole set forth in the (6) above, wherein the water content of the aqueous polyhydric alcohol solution is in the range of 30 to 70% by weight.

The objects can be further accomplished by (8) an insole set forth in any of the (1) to (7) above, wherein the thickness of the flexible resin sheet is in the range of 0.3 to 1.0 mm.

The objects can be further accomplished by (9) an insole set forth in any of the (1) to (8) above, wherein the flexible resin material of the flexible resin sheet is at least one member selected from the group consisting of polyurethane, polyvinyl chloride, and an irradiated ethylene-vinyl acetate copolymer.

The objects can be further accomplished by (10) an insole set forth in the (9) above, wherein the vinyl acetate content of the irradiated ethylene-vinyl acetate copolymer is in the range of 5 to 30% by weight.

The objects can be further accomplished by (11) an insole set forth in the (9) or (10) above, wherein the irradiation is carrier out with electron rays or gamma rays.

The objects can be further accomplished by (12) an insole set forth in any of the (9) to (11) above, wherein the irradiation dose is in the range of 4 to 10 Mrad.

The objects can be further accomplished by (13) an insole set forth in any of the (9) to (12) above, wherein the degree of cross-linking of the irradiated ethylene-vinyl acetate copolymer is in the range of 70 to 95%.

The objects can be further accomplished by (14) an insole set forth in any of the (1) to (13) above, wherein a cushioning layer is provided on the surface of the tightly sealed bladder which contacts with a foot sole.

The objects can be further accomplished by (15) an insole set forth in any of the (14) above, wherein the cushioning layer is formed of at least one member selected from the group consisting of a woven fabric, a non-woven fabric, a raised fabric, a knitted fabric, a vinyl leather, a natural leather and a synthetic leather.

The objects can be further accomplished by (16) an insole set forth in any of the (1) to (5) above, wherein an insole-fixing material is provided on the surface of the tightly sealed bladder which contacts with the surface of the shoe sole.

The objects can be further accomplished by (17) an insole set forth in the (16) above, wherein a peelable and pressure-sensitive adhesive tape is used as the insole-fixing material.

The objects can be further accomplished by (18) an insole set forth in the (16) or (17) above, wherein the insole-fixing material comprises a releasable pressure-sensitive adhesive layer, a substrate, and a releasable pressure-sensitive adhesive layer.

The objects can be further accomplished by (19) an insole set forth in the (16) or (17) above, wherein the insole-fixing material comprises a releasable pressure-sensitive adhesive layer, a substrate, and an adhesive layer.

The objects can be further accomplished by (20) an insole set forth in any of the (1) to (15) above, wherein the tightly sealed bladder comprises a central part and a heel part, provided in that a shoe sole is divided into a toe tip part, central part and a heel part.

The objects can be further accomplished by (21) an insole set forth in the (20) above, wherein an insole-fixing material is provided on the surface of the tightly sealed bladder which contacts with the surface of the shoe sole.

The objects can be further accomplished by (22) an insole set forth in the (21) above, wherein a peelable and pressure-sensitive adhesive tape is used as the insole-fixing material.

The objects can be further accomplished by (23) an insole set forth in the (21) or (22) above, wherein the insole-fixing material comprises a releasable pressure-sensitive adhesive layer, a substrate, and a releasable pressure-sensitive adhesive layer.

The objects can be further accomplished by (24) an insole set forth in the (21) or (22) above, wherein the insole-fixing material comprises a releasable pressure-sensitive adhesive layer, a substrate, and an adhesive layer.

As mentioned above, in the insole of the present invention, a protruded part is formed in a conventional shoe sole-shaped insole or such an insole as comprising only a central part of a conventional shoe sole-shaped insole which corresponds to the arch part thereof to so as to produce an arch on a flatfoot sole or to stimulate the entire area of the arch. When this protruded part is placed inside a shoe so that the protruded part should press a whole arch, namely a portion between a sole and a lateral side of a foot, most of a liquid is transferred to and concentrated on the protruded part during the exertion of a load on a toe tip and a heel thereby pressing the entire portion corresponding to the arch. By this action, an arch can be formed even for a flatfooted person similar to that of a normal person, and the flatfoot can be easily corrected without any pains nor fatigue. Further, for healthy persons having arches, the protruded part can be excellently fit for a shape of an arch by transferring the liquid filled in the tightly sealed bladder thereto and changing its shape flexibly independent on the size and shape of the arch. Furthermore, a suitable pressing force which is equivalent to that by stepping (on a green bamboo) can be imparted to an arch. At the same time, since the liquid filled in the insole is transferred to portions other than the protruded part during the movement of foot or walking, the pressing force is not continuously exerted only on an arch and therefore the insole of this invention can be used over a long period. Further, the pressing force exerted on the arch can be automatically controlled by utilizing a load exerted on a toe tip part and a heel part or a surrounding part of the arch thereof from a foot sole, and changing the shape of the protruded part so as to fit for a shape of an arch of an individual of himself. On other words, a flatfoot can be easily corrected by forcibly forming an arch with a developed state on a sole of a flat-footed person. For normal persons, the entire arch can be stimulated, a satisfactory pressing force obtainable by the exercise of stepping on a green bamboo can be suitably applied to the arch, and an excellent massaging effect can be obtained.

When an insole comprises only a central part which corresponds to an arch part of the shoe-shaped insole, such a defect as of making the inside space of the shoe (particularly when high-heeled shoes or boots used) too tight to make the shoes put in with much difficulties and to hurt the user's feet can be overcome.

When the insole of the present invention is used only for the central part which corresponds to the arch part of shoe sole, this type of the insole has such an advantage as that it can be used for shoes with all sizes, because the width of the arch part of the shoe insole is not largely changed as compared with the length thereof depending on various sizes of shoes, and such insole is hardly moved transversely by providing an insole-fixing material on the surface thereof which contacts with a shoe sole.

Further, by using polyhydric alcohol as the liquid, the insole of this invention having durability, preventing loss of liquid even after the prolonged use thereof, and giving comfortable pressing force to the whole area of foot sole without any freezing during the actual use can be obtained.

Furthermore, in this invention, by simultaneously providing a cushioning layer on the surface of the tightly sealed bladder which contacts with a foot sole, the insole can release or alleviate fatigue of feet and waist by continuous standing, give excellent feeling to foot soles, and realize improvement of absorbency to make foot soles less humid.

### BRIEF DESCRIPTION OF THE DRAWINGS

FIG. 1 is a schematic top plan view illustrating a left insole as one embodiment of the present invention.

FIG. 2 is a perspective view schematically illustrating the insole of Fig. 1 placed in a shoe.

FIG. 3 is a schematic perspective diagram of a insole showing the movements of a load exerted on a peripheral part of an arch and a liquid filled in the insole during the actual use thereof.

FIG. 4 is a schematic top plan view illustrating a insole as another embodiment of the present invention.

FIG. 5 is a schematic top plan view illustrating a insole as a further embodiment of the present invention.

FIG. 6 is a schematic top plan view illustrating a conventional insole as one typical embodiment.

FIG. 7 is a bottom plan view of FIG. 6.

FIG. 8 is a cross section taken along line A-A of FIG. 6, showing the practical use of the insole of FIG. 6 placed in a shoe.

### DESCRIPTION OF THE PREFERRED EMBODIMENT

This invention will be described more specifically with referring to the drawings as follows.

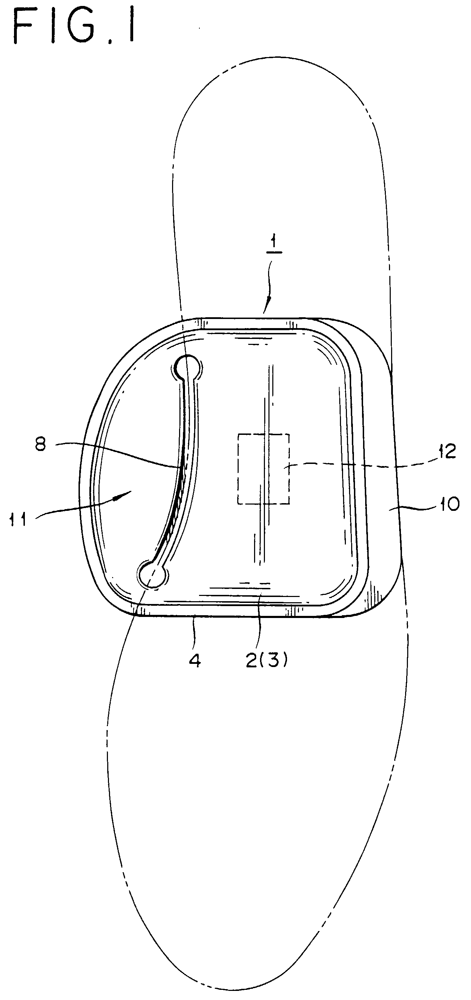

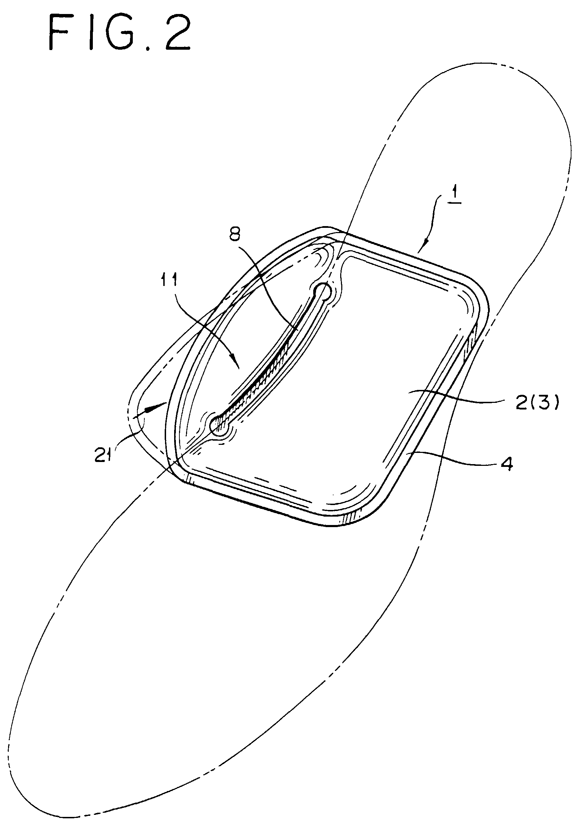

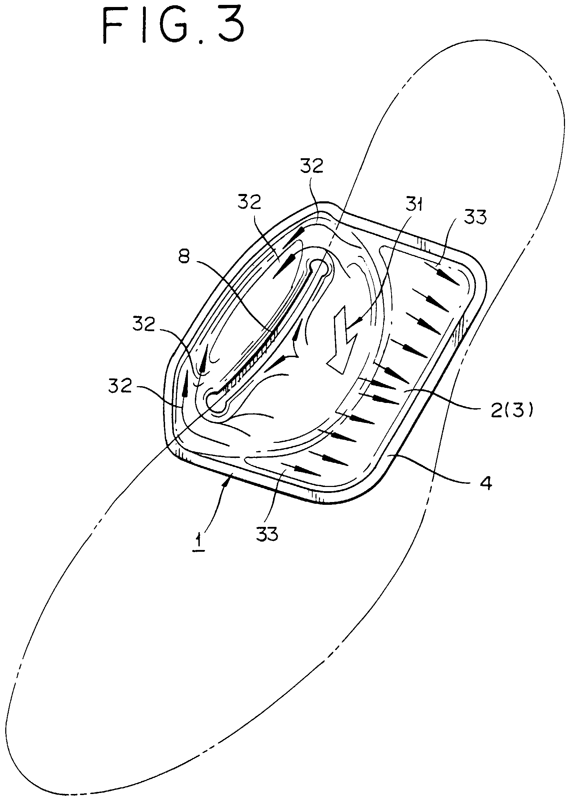

FIG. 1 is a schematic top plan view illustrating a left insole as one embodiment of the present invention. FIG. 2 is a perspective view schematically illustrating the insole of Fig. 1 placed in a shoe. FIG. 3 is a schematic perspective diagram of a insole showing the movements of a load exerted on a peripheral part of an arch and a liquid filled in the insole during the actual use thereof.

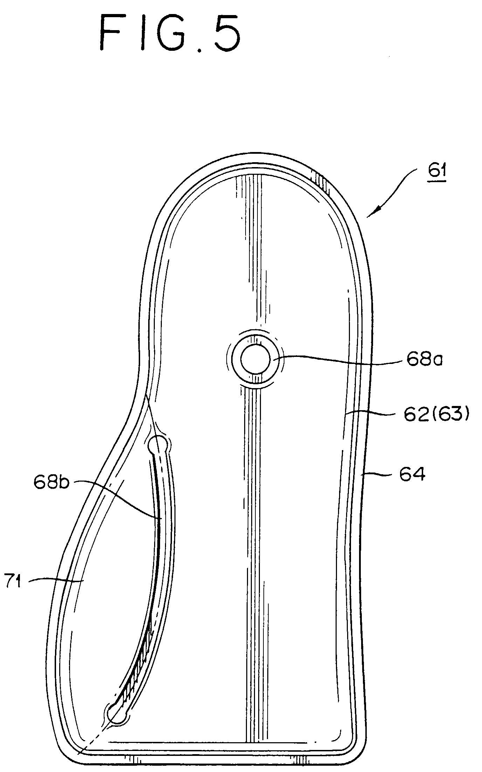

As shown in Figs. 1 to 3, a insole 1 as one embodiment according to this invention, comprises a tightly sealed bladder formed of two flexible resin sheets 2(3) (hereinafter, numbers in parentheses shown in Figures are not shown because they are similar to the conventional insoles shown in Figs. 5 to 7) having the circumference parts 4 thereof fused, and a liquid filled overall in the bladder to such an extent as that the two sheets of the bladder may partially come into mutual contact when a load is exerted on the interior of this bladder. In this embodiment, the tightly sealed bladder is formed of only a central arch part which corresponds to an arch part of a shoe sole provided that the shoe sole is divided into three parts, i.e. a toe tip part, a central arch part and a heel part, and a protruded part 11 (hatched part in FIG. 1). This protruded part 11 is formed as a part which can satisfy such a function as ① a function for correcting a flatfoot by forming an arch on a flatfoot sole with a pressing force of a liquid to be generated when a load is exerted on a peripheral part of the arch in actual use, and ② a function for promoting health by simulating an entire arch of a normal person with a pressing force obtainable by the exercise of stepping and generated when a load is exerted on a peripheral part of the arch in actual use.

In the tightly sealed bladder, a barrier 8 against a liquid (5) is preferably formed at any part other than the circumstance parts 4 thereof (mainly at the central part thereof) by partially fusing the two sheets 2(3). In this case, by providing a barrier 8 at the border position between the shoe sole part and the protruded part 11 as shown in FIG. 2, the insole can be folded easily by using such barrier 8, as shown by an arrow mark 21 in FIG. 2. The insole with such barrier has also has an advantage of easily deciding the position of the insole 1 to be placed in a shoe. In this case, however, an attention must be paid so that the length of the barrier 8 should be less than the entire length of the border line between the shoe sole and the protruded part 11. To be more specific, if the barrier 8 is divided the inner space of the insole into a shoe sole part and a protruded part, the movement of the liquid filled in the inner space is unduly limited, causing insufficient pressing force to be exerted on an arch in use. The formation of the barrier 8 may constitute an obstacle in flowing the liquid (5) within the tightly sealed bladder with a load 31 exerted on the insole 1 as shown in FIG. 3, to make the flow of the liquid (5) slower because of the roundabout flow thereof as shown with an arrow mark 32 in FIG. 3, and to stimulate other parts of a foot sole with the pressing force. Further, the barrier 8 can also supplement and increase the total fused area because of the fused part of the barrier in addition to that of the circumstance part 4, and therefore, impart an effect of preventing breakage to the tightly sealed bladder.

As shown in FIG. 1, a fused extra part 10 may be further formed in width of a shoe as continuous to the outer side of the circumference part 4, so that the insole can be used for the maximum size thereof. Such fused extra part can be cut partially for the adjustment to the size of the used shoe. This method is effective to prevent widthwise slip of the insole 1. In order to effectively prevent not only lengthwise slip but also widthwise slip, it is desirable to provide a insole-fixing material 12 on the shoe-sole side of the sheet (3).

When the fused extra part 10 of the pair of sheets 2(3) (or the circumstance part 4) is formed in the shape of a shoe sole, the edge of the part is desirable to be round like shown in FIG. 1, because the sharp edge may hurt feet.

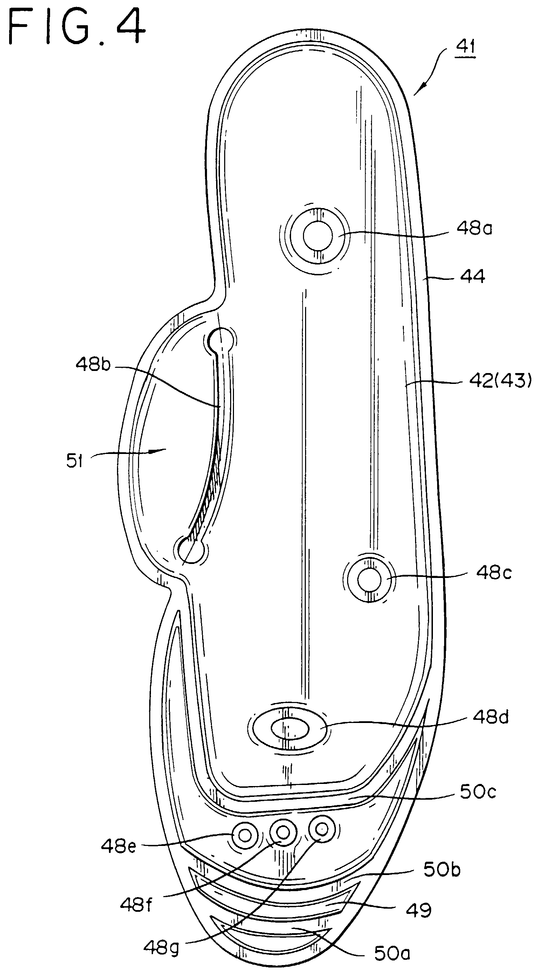

FIG. 4 is a schematic top plan view illustrating a insole as another embodiment of the present invention.

An Insole 41 comprises a tightly sealed bladder formed of two flexible resin sheets 42(43) in the shape of a shoe sole having the circumference parts 44 thereof fused, and a liquid partially filled in the bladder to such an extent as that the two sheets of the bladder may partially come into mutual contact when a load is exerted on the interior of this bladder. In this embodiment, the insole further comprises a protruded part 51 as a part projected from the shoe sole-shaped bladder which can satisfy such a function as ① a function for correcting a flatfoot by forming an arch on a flatfoot sole with a pressing force of a liquid to be generated when a load is exerted on a peripheral part of the arch in actual use, and ② a function for promoting health by simulating an entire arch of a normal person with a pressing force obtainable by the exercise of stepping and generated when a load is exerted on a peripheral part of the arch in actual use.

In the tightly sealed bladder, barriers 48a to 48g against the liquid (45) are preferably formed at any parts other than the circumstance parts 4 thereof (mainly at the central part thereof) by partially fusing the two sheets 42(43). The effect obtained by providing barriers 48a to 48g is similar to that obtained by the barrier 8 in the insole 1.

In the toe tip part 49 of the two sheets 42(43), fused extra parts 50a to 50c may be further formed as continued to the circumference part 44 in addition to the most outer peripheral part thereof, and the inner part as far as the most inner fused part 50c is used as the inner space of the tightly sealed bladder (which is a part filled with a liquid), as shown in FIG. 4. For the purpose of obtaining a shoe sole having its shape fit for the shoe size used, the fused extra part 50a or 50b are designed so as to cut the insole along the border thereof (e.g., to make the length so far as the circumstance 4 of 25.0 inches, and that so far as the fused part 50a of 24.5 inches and 50b of 24.0 inches).

FIG. 5 is a schematic top plan view illustrating a insole as a further embodiment of the present invention. An insole 61 with this constitution can be formed by removing the toe tip part from the insole 41 shown in FIG. 4 to form the part between the arch part and the heel part of the insole 41. In other words, the insole 61 comprises a central part and a heel part, provided that a shoe sole is divided into three parts, namely a toe tip part, a central arch part and a heel part. The insole 61 comprises a tightly sealed bladder formed of two flexible resin sheets 62(63) having the circumference parts 64 thereof fused, and a liquid partially filled in the bladder to such an extent as that the two sheets of the bladder may partially come into mutual contact when a load is exerted on the interior of this bladder. In this embodiment, the insole 61 further comprises a protruded part 71 from the half shoe sole-shaped insole which can satisfy such a function as ① a function for correcting a flatfoot by forming an arch on a flatfoot sole with a pressing force of a liquid to be generated when a load is exerted on a peripheral part of the arch in actual use, and ② a function for promoting health by simulating an entire arch of a normal person with a pressing force obtainable by the exercise of stepping and generated when a load is exerted on a peripheral part of the arch in actual use.

The insole of this invention having a constitution as shown in Fig. 5 has such advantages as that the position of the insole to be placed in a shoe can be more easily decided and the insole can be placed in the desired place of a shoe more easily as compared with the insole as shown in Fig. 1, that the insole can be prevented from moving during the walking because it is fixed with the heel part thereof, and further that the impact generated on a heel part when shoes, in particular high-heeled shoes are put on can be absorbed.

In accordance with this embodiment, an insole-fixing material may be provided on the surface of the heel part which contacts with the surface of the shoe sole. By providing an insole-fixing material on the surface of the heel part which contacts with the surface of the shoe sole, such advantages can be obtained as that it is not remained in the shoe sole and the shoes can be used intact with the exchange of the insole kept easy when shoes is to be used after removing the insole, that the insole can be more effectively prevented from moving during the walking, and further that the impact generated on a heel part when shoes, in particular high-heeled shoes are put on can be absorbed to a larger extent.

The insole-fixing materials which are usable in this invention are not particularly limited, as far as that they can fix the insole on a shoe sole so as to prevent the move in the lengthwise and widthwise slip thereof. They are varied with the shape of the used insole, for example. As examples thereof, various commonly available adhesives and pressure-sensitive adhesives may be cited. Releasable pressure-sensitive adhesive materials may be preferably used. As typical examples thereof, double coated tapes, materials comprising a releasable pressure-sensitive adhesive layer, a substrate and a releasable pressure-sensitive adhesive layer, and materials comprising a releasable pressure-sensitive adhesive layer, a substrate and an adhesive layer may be cited. In this invention, for insoles shown in Fig. 1, for example, double coated tapes having a relatively strong adhesive force may be preferably used, while for insoles shown in Fig. 4 or Fig. 5, materials comprising a releasable pressure-sensitive adhesive layer, a substrate and a releasable pressure-sensitive adhesive layer, and materials comprising a releasable pressure-sensitive adhesive layer, a substrate and an adhesive layer which have a relatively weak adhesive force may be preferably used, in addition to the double coated tapes. Although the kinds of the adhesives or pressure-sensitive adhesives which are used for the insole side tightly sealed bladder are not particularly restricted, the adhesive or pressure-sensitive adhesive to be used for the shoe sole side is preferably a releasable pressure-sensitive adhesive.

When the insole-fixing material has a three layer structure consisting of a releasable pressure-sensitive adhesive layer, a substrate and an adhesive layer, the releasable pressure-sensitive adhesive layer is provided on the surface of the heel part which contacts with the shoe sole, and the adhesive layer is provided on the surface of the heel part which contacts with the tightly sealed bladder. By this constitution, the insole can be repeatedly used with this releasable pressure-sensitive adhesive layer, even when the insole is exchanged to another shoe.

The substrates which can be used in this invention are not specifically limited, so far as they can be used in the coating process. As examples thereof, such fabrics as a woven fabric, a non-woven fabric, a raised fabric, a knitted fabric, a vinyl leather, a natural leather and a synthetic leather, a cellulose film, plastic films such as of polyethylene terephthalate (PET), polyvinyl chloride (PVC), ethylene-vinyl alcohol copolymer (EVOH), styrene-butadiene copolymer, polypropylene, polyolefin, polyester, polyamide, polyimide, polystyrene, polyurethane, polycarbonate, polyvinylidene chloride, polyacrylic ester, epoxy resin, 4-fluorinated ethylene, ethylene-vinyl acetate copolymer, vinyl chloride-acrylic ester copolymer, vinyl chloride-vinyl acetate copolymer, polyvinyl butyral, acrylonitrile-acrylic ester copolymer, fluorine-containing polymer, and silicone type polymer and silicon-containing polymer, metal foil may be used depending on its application and purpose.

The pressure-sensitive adhesives which forms a releasable pressure-sensitive adhesive layer to be used in this invention are not particularly restricted so far as that the pressure-sensitive adhesive force aimed at can be obtained. As typical examples thereof, polyurethane type pressure-sensitive adhesives such as of thermally cross-linked polyurethane, rubber type pressure-sensitive adhesives such as of natural rubber (polyisoprene), styrene-butadiene rubber, butyl rubber, silicone rubber, polyisobutylene, styrene-isoprene-styrene block copolymer, styrene-butadiene-styrene block copolymer, styrene-ethylene-styrene block copolymer, and ethylene propylene rubber, ethylene type pressure-sensitive adhesives such as of ethylene-vinyl acetate copolymer, ethylene-ethyl acrylate copolymer, ethylene-vinyl acetate-methacrylic acid copolymer, partially saponified ethylene-vinyl acetate, acrylate type pressure-sensitive adhesives obtained by copolymerizing (meth)acrylate alkyl ester as a main component with such a monomer as (meth)acrylonitrile, (meth)acrylic acid, and styrene, butyl polyacrylate, 2-ethylhexyl polyacrylate, polyacrylic acid, and polyvinyl butyl ether may be used singly or in a mixture of two or more members cited above.

The pressure-sensitive adhesive layer may be formed by introducing into the pressure-sensitive adhesive as just mentioned above a tackifier such as polymerized rosin, rhodinic ester such as rhodinic acid glycol ester, rhodinic acid glycerol ester, and rhodinic acid pentaerythritol ester, and the hydrogenolyzed products thereof, rhodinic acid such as rhodinic acid phenol alkyl phenol formaldehyde resin, and the derivatives thereof, and terpene resin, phenol resin, and coumarone-indene resin.

The adhesives which forms the adhesive layer to be used in this invention are not specifically limited so far as that the adhesive force aimed at can be obtained. As typical examples thereof, acrylate type adhesives, aromatic polymer adhesives, styrene-butadiene rubber type adhesives, butyl rubber type adhesives, cellulose ester, cellulose ether, cyanoacrylate type adhesives, rubber (elastomer) type adhesives, epoxy resin type adhesives, epoxy-polyamide, epoxy-nylon, epoxy-polysulfide, epoxy-elastomer type adhesives, ethylene-ethyl acrylate copolymer type adhesives, ethylene-vinyl acetate copolymer type adhesives, furan resin type adhesives, melamine rein type adhesives, chloroprene rubber type adhesives, chloroprene rubber-phenolic type adhesives, nitrile rubber type adhesives, nitrile rubber-phenolic type adhesives, nylon type adhesives, phenol resin type adhesives, phenolic-epoxy, phenoxy resin, polyamide, polybenzimidazole, polyester, polyimide, polyvinyl chloride and the copolymers thereof, polysulfide type adhesives, polysulfone type adhesives, polyurethane type adhesives, polyvinyl acetal, polyvinyl acetate, polyvinyl ether, and polyvinyl alcohol may be selectively used depending the kind of the used sheet, for example.

In accordance with this invention, the amount of the releasable pressure-sensitive adhesive or adhesive to be coated on the substrate may be such that the thickness of the insole-fixing material becomes in the range of 0.2 to 2 mm, preferably 0.5 to 1 mm.

In the tightly sealed bladder, a barriers 68b is desirably formed along the inner border of the shoe sole-shaped insole without connecting to the circumstance part 64 by partially fusing the two sheets 62(63). Further, a barrier 68a maybe additionally or alternatively formed approximately in the heel part of the tightly sealed bladder. The effect obtained by providing barriers 68a and 68b is similar to that obtained by the barrier 8 in the insole 1.

When a person places the insoles of this invention having a constitution shown in each embodiment as described above, for example as shown in FIG. 3, and stands up, the liquid 5 is transferred into the protruded part 11 which is projected from the central part of the insole 1 as shown with an arrow mark 32 in FIG. 3 with the load 31 exerted on the surrounding part of the arch, to expand the protruded part 11 (the arch part). In this case, the protruded part 11 is expanded also by a load exerted on the toe tip part and the heel part of the insole 41. By its mechanism, even for flat-footed persons who have almost no arches, the flatfoot can be corrected by pressing from the foot sole side a part which corresponds to an arch of a normal person, forming forcibly an arch and stimulating suitably the arch part. Further, since this insole has also ample massaging effect, a flatfoot can be corrected by the use of the insole 1 without any pains. For normal persons, since the protruded part 11 imparts the stimulation equivalent to that obtained by stepping to an entire arch and suitably stimulates effective spots, a satisfactory massaging effect can be obtained and the promotion of health can be attained. In this case, while with a conventional flatfoot-correcting device, a part corresponding to an arch is continuously stimulated, with the insole 1 of this invention, since the liquid 5 filled in the insole 1 is constantly moved in the opposite directions (as shown with an arrow mark 33 in FIG. 3) by moving or walking, the protruded part 11 is not always continued of stimulating a part corresponding to an arch. The problem on the continuous use of an insole can be solved. Further, when a flexible material is solely used as in conventional insoles or flatfoot-correcting devices, a pressing force equivalent to that by stepping have not been produced. By the insole 1 according to this invention, a pressing force equivalent to that by stepping can be obtained by suitable viscosity and elasticity of the liquid 5. By placing an arch-shaped flexible material, not only a desired pressing force can be imparted to an arch part, but also such a defect as that medical effects demanded for the correction of flatfoot are not manifested satisfactorily at all can be overcome.

Further, the Insole 1 as one embodiment of this invention has such advantages as that it can be produced at a lower cost than the insoles 41 and 101 having a shape of a shoe sole, can attain both effects of correcting flatfoot and of promoting health by means of a suitable pressing force, both of which are characteristics of this invention. Furthermore, the insole of this invention can be produced at low cost. Since the lengthwise and widthwise slip thereof can be prevented by proving an insole-fixing material 12 on the insole 1 as shown in FIG. 1, diversity of insole sizes along with shoe sizes is not necessary, and this insole can be applied to any size of shoes. Since the insole 1 has no toe tip and heel parts on which a load by a foot tends to be exerted and only an arch part on which a relatively small load is exerted is used for the insole, such defects by commonly available shoe sole-shaped insoles as that the user's foot in the shoe (particularly, the size in the direction of the foot instep) is made tight, such shoes are put on with much difficulties and a pain is generated to the foot can be also overcome.

The constituting elements of the insole of this invention will be described as blow.

The thickness of the flexible resin sheet which can be used in the insole of this invention is usually in the range of 0.3 to 1.0 mm, preferably in the range of 0.4 to 0.8 mm and more preferably in the range of 0.45 to 0.7 mm. If the thickness is less than 0.3 mm, the durability thereof is unduly deteriorated because of the insufficient mechanical strength. Conversely, if it is more than 1.0 mm, the insole becomes too hard to impart a sufficient pressing force to foot soles due to the lack in the liquid flow, and the produced insole becomes unduly expensive.

Both or either one of the inner side of the above-mentioned two flexible resin sheets are preferably subjected to the embossing. By such embossing, stickiness of the two sheets can be avoided and the operation for injecting with a liquid, particularly viscous liquid can be performed easily.

For the flexible resin material of the above-mentioned flexible resin sheet, at least one member selected from the group consisting of polyurethane, polyvinyl chloride and irradiated ethylene-vinyl acetate copolymers may be desirably cited in terms of durability, low possibility of leaking a liquid (non-permeability of liquid) and economy.

When a irradiated ethylene-vinyl acetate copolymer is to be used as the material for the flexible resin sheet, the vinyl acetate content thereof is in the range of 5 to 30% by weight, preferably 8 to 20% by weight. The number-average molecular weight thereof is generally in the range of 10,000 to 150,000, preferably 30,000 to 100,000. If the vinyl acetate content thereof is less than 5% by weight or the number-average molecular weight thereof is less than 10,000, the produced flexible resin sheet is deficient in flexibility and sealing ability. On the other hand, if the vinyl acetate content thereof is more than 30% by weight or the number-average molecular weight thereof is more than 150,000, the sheets become too soft to attain the objects of this invention. Further, for the purpose of obtaining the irradiated vinyl acetate copolymer having such conditions as described above, the irradiation dose is usually in the range of 4 to 10 Mrad, preferably 5 to 8 Mrad in a nitrogen atmosphere at 20 °C. If the irradiation dose is less than 4 Mrad, the cross-linking reaction of non-cross-linked copolymer of ethylene and vinyl acetate as a subject to be irradiated is carried out insufficiently, and the decrease of the liquid-leaking ratio can not be attained. Conversely, if it exceeds 10 Mrad, the degradation of the copolymer is started to make the material difficult to use as an insole. The subjects to be irradiated include, for example, a tightly sealed bladder which has been formed by fusing, as well as materials which will be formed into a tightly sealed bladder. As typical examples of the latter case, powers and pellets of non-cross-linked copolymers, and sheets to be formed from the non-cross-linked copolymer may be cited. Electron rays, γ ray, or the combination thereof can be used as the source of this radiation. As the source of γ ray, Co-60 gamma ray may be used and as the electron beam source, a resonance transformer may be used. Although electron rays have such advantages as a high cross-linking speed, high energy efficiency, no heat to be exposed on the material to be irradiated, rapid startup of facility and easy control of production conditions, as well as small variability of the quality of the irradiated material, they have such problems as of the limitation of transmitting length, and of the unsuitability for a thick material. Therefore, the kind of the used source for irradiation must be chosen suitably depending on various conditions such as the thickness and kind of material to be irradiated. The cross-linking degree of the irradiated ethylene-vinyl acetate copolymer is desirable to be adjusted generally within the range of 75 to 90% by suitably controlling the above-mentioned irradiation conditions. If the cross-linking degree is less than 70%, the strength of the irradiated copolymer is insufficient, the durability thereof is degraded, and therefore, there are some possibilities of disadvantageously breaking a part of the tightly sealed bladder thereby leaking the liquid filled in the tightly sealed bladder therefrom in use. On the other hand, if it exceeds 95%, although the strength of the irradiated copolymer is sufficient, the deformation along arch shapes of insoles becomes difficult because of the inferior flexibility of the produced sheets.

The liquids which can be filled in the insoles according to the present invention are not particularly limited. They include, for example, water, polyhydric alcohols, and aqueous polyhydric alcohol solutions. At least one member selected among polyhydric alcohols, and aqueous polyhydric alcohol solutions may be preferably used in terms of no possibilities of decreasing the amount of the liquid filled in the bladder even after the protracted use and of freezing in actual use. As examples of the polyhydric alcohol which can be used in this invention, ethylene glycol, diethylene glycol, triethylene glycol, tetraethylene glycol, polyethylene glycol, propylene glycol, dipropylene glycol, 1,3-butylene glycol, polypropylene glycol, neopentyl glycol, 2,2,4-trimethyl-1,3-pentanediol, 1,4-cyclohexane dimethanol, glycerin, pentaerythritol, dipentaerythritol, tripentaerythritol, threitol, neopentyltol, ribitol, allitol, galactitol, xylitol, sorbitol, mannitol, trimethylol ethane, trimethylol propane, and enneaheptytol anhydride may be cited. Among other polyhydric alcohols cited above, propylene glycol and glycerin may be preferably used in terms of appropriate viscosity, non-odorous liquid (gel), and such physical properties as non-volatilizability and low solidifying points (non-freezing property), and further such characteristics as antiseptic ability and bactericidal ability, and easy access at low costs. Further, into the polyhydric alcohol, such an antifumigating agent as tin compounds and copper compounds may be introduced.

Alternatively, an aqueous polyhydric alcohol solution may be preferably used as the liquid. In this case, although the water content in the aqueous polyhydric alcohol solution is not particularly limited, it is preferably in the range of 30 to 70% by weight. The aqueous polyhydric alcohol solution has such advantages as of being capable of easily adjusting an approximate viscosity.

The amount of the liquid filled in the tightly sealed bladder is such an extent as that at a part where a high load is exerted, the sheets come to mutual contact each other, while to a part where a low load is exerted, the liquid can move. If the liquid is filled in the tightly sealed bladder to such an extent as that even at the part where a maximum load is exerted, the liquid is present between the two sheets, the liquid is constantly present on the entire foot sole of the user, which undesirably causes fatigue to the user after using for a long time.

In the insole of this invention, it is possible to attach a cushioning layer to the surface of the tightly sealed bladder which contacts with the foot sole, if occasion demands. Such cushioning layer and the liquid filled in the bladder can supplement almost the cushioning function for such persons as nurses, employee at department stores or hotels, and assembly workers at auto makers who must continue standing on such a solid floor as of concrete and marble over a long period and have a load continuously exerted on their cartilage such as of knee joints having a cushioning function, or aged persons who have lowered cushioning function. Further, with this cushioning layer provided in the insole, such symptoms as swollen feet and lumbago because of the degradation of systemic circulation of blood, and overload on his legs and waist can be eliminated or alleviated. Furthermore, by using a material with excellent hygroscopicity in the cushioning layer, components such as sweat perspired from foot soles in use can be rapidly absorbed, and comfortable and less humid feeling of foot soles can be maintained even after the prolonged use.

As the materials used in the cushioning layer, any materials which are effective to decrease the impact of a person's foot may be used. As preferable examples thereof, at least one member selected from the group consisting of woven fabrics, non-woven fabrics, raised fabrics, knitted fabrics, vinyl leathers, natural leathers and synthetic leathers may be cited. In the case of the natural or synthetic leather, suede type leathers are particularly preferably used. In the case of the non-woven fabric, embossed non-woven fabrics are desirably used. Among other materials cited above, suede type natural and synthetic leathers, non-woven fabrics and raised fabrics are most preferable. These materials, with their excellent impact absorption capacity and liquid absorbency, can not only eliminate or alleviate fatigue of legs and waist occurred from long standing but also keep a foot sole comfortable and less humid.

The thickness of the cushioning layer should be suitably decided based upon the material thereof and the user's weight. It is generally in the range of 0.01 to 2 mm. In consideration of the elimination or alleviation of fatigue of legs and waist occurred by long standing, as well as in terms of keeping a foot sole comfortable, increasing the hygroscopicity and repressing the humidity of feet, it is desirably in the range of 0.5 to 1 mm. If the thickness of the cushioning layer is more than 2 mm, the inner space of shoes is too small and foot soles are unduly compressed. With respect to the size (shape) of the cushioning layer, although it may be roughly the same as that of the insole, it is needless to say that the cushioning layer can be used only for the part of the insole such as the toe tip part and the heel part, on which a comparatively large load is exerted.

The means of attaching the cushioning layer are not specifically limited. As example thereof, a method which comprises applying a suitable adhesive on either or both of the sheet cut in the desired shape and the cushioning layer after the formation into the tightly sealed bladder and prior to the filling with the liquid, and attaching the sheet with the cushioning layer may be used. Alternatively, a method which comprises subjecting the foot sole side sheet and the cushioning layer to the lamination by coextruding prior to the formation into the tightly sealed bladder to form a laminate of the two components, and cutting the laminate in the desired shape.

The insole-fixing materials which are usable in this invention are the same as defined in the insole shown in Fig. 5.

The insole of this invention as mentioned above can be produced by the following procedure, for example. It, however, should be noted that the method for the production of the insole of the present invention is not limited by the methods as described below and the conventional techniques for the production and processing which are relevant thereto can be suitably utilized and applied.

Two flexible resin sheets having a desired shape which have been superposed are placed on a bottom die provided with heating parts corresponding respectively to the circumstance, a barrier(s), and optionally fused extra part(s), and having a width of about 5 mm, for example. A top die is placed thereon and the pair of dies is heated with high frequency, ultrasonic wave, or other heating means, thereby fusing the above-mentioned parts. For example, a tightly sealed bladder is formed by high-frequency fusing the two sheets with a maximum current in the range of 1.5 to 1.25 A, under a pressure in the range of 1.5 to 3 kg/cm², and for an oscillation time in the range of 4 to 10 seconds. In this sealing, some part of the circumference part must be left unsealed. When an irradiated ethylene-vinyl acetate copolymer is used as the flexible resin material for the flexible resin sheet, for example, the whole tightly sealed bladder is irradiated in a nitrogen atmosphere at 20 °C by using gamma ray or electron rays as the irradiation source with an irradiation dose in the range of 4 to 10 Mrad, to form a tightly sealed bladder in which the un-cross-linked copolymer has been cross-linked. In the case of optionally providing a cushioning layer, a suitable adhesive may be applied to either or both of the sheet and the cushioning layer after the formation into the tightly sealed bladder and prior to the filling with the liquid, and thereafter the sheet and the cushioning layer may be attached each other. Alternatively, the film sheet of the foot sole side and the cushioning layer are laminated by coextrusion prior to the formation into the tightly sealed bladder thereby forming a laminate, and thereafter the formed laminate is cut in the desired insole shape and subjected to the fusing operation to form a tightly sealed bladder.

Subsequently, in the obtained tightly sealed bladder , a specific amount of the liquid is filled from the unfused part as mentioned above, and thereafter, the unfused part is sealed in the similar manner as of the above-mentioned procedure. Thus, the insole of this invention can be obtained. When the irradiated ethylene-vinyl acetate copolymer is used as the flexible resin material, an uncross-linked ethylene-vinyl acetate copolymer may be interposed between the unfused parts and thereafter sealed, to form the insole of this invention.

When an insole-fixing material is to be provided, for example, a peelable pressure-sensitive adhesive double coated tape having a size suitable as the insole-fixing material may be optionally attached to the foot sole side surface of the tightly sealed bladder prior to use and the insole is placed on a shoe sole to fix the insole on the shoe sole with the tape by means of a foot pressure.

The present invention will be illustrated more specifically with referring to the following working example.

### Example 1

A pair of ethylene-vinyl acetate copolymer sheets 2(3) having the vinyl acetate content of 10% by weight, the number-average molecular weight of about 50,000, and the thickness of 0.45 mm was cut in the shape of a shoe sole which comprises a central part [provided that the shoe sole is divided into three parts, i.e. a toe tip part, a central arch part and a heel part], and a protruded part 11 from the shoe sole, as shown in FIG. 1. The insole having this shape had such a function as ① for correcting a flatfoot by forming an arch on a flatfoot with a pressing force of a liquid to be generated when a load is exerted on the toe tip part and the heel part thereof in actual use, or ② for promoting health by simulating an entire arch of a normal person with a pressing force obtainable by the exercise of stepping and generated when a load is exerted on the toe tip part and the heel part thereof in actual use. After cutting the sheets in the above-mentioned shape, as shown in FIG. 1, the circumference part 4 and barrier part 8 of the sheets 2(3), except for a portion of the heel side thereof, were fused in a fusing width of about 5 mm by means of high-frequency fusing with a maximum current of 1.20 A and for an irradiation time of 6 seconds, to form a tightly sealed bladder.

To the thus obtained tightly sealed bladder, 8 Mrad of cobalt-60 as a source is exposed in a nitrogen atmosphere at 20 °C. In this case, the cross-linking degree of the irradiated ethylene-vinyl acetate copolymer was 85%.

Then, about 20 ml of propylene glycol was introduced as a liquid (5) in the produced tightly sealed bladder from the unfused part thereof. Thereafter, this unfused part was high-frequency fused under the same conditions as mentioned above.

A cushioning layer (6) composed of a suede type synthetic leather having a roughly same shape as of the obtained insole 1 was attached to the sheet 2 side thereof (the surface contacting with the shoe sole) with chloroprene rubber (produced by Denki Kagaku Kogyo K.K. under product code "M-120 type"), to obtained an insole 1a.

### Example 2

The procedure of Example 1 was repeated except for introducing an aqueous propylene glycol solution (concentration of 50% by weight) in place of propylene glycol, to obtain an insole 1b.

### Example 3

An insole 1c was obtained by the same manner as of Example 1 except for introducing glycerin instead of propylene glycol.

### Example 4

The procedure of Example 1 was repeated except that glycerin was introduced in place of propylene glycol and the use of the cushioning layer (6) of suede type synthetic leather and with a roughly same shape of the insole was omitted, to obtain an insole 1d.

### Example 5

An insole 1e was obtained by the same manner as of Example 1 except that a pair of polyurethane sheets 2(3) with a thickness of 0.6 mm was used instead of the ethylene-vinyl acetate copolymer sheets 2(3) having the vinyl acetate content of 10% by weight, the number-average molecular weight of about 50,000, and the thickness of 0.45 mm, and the tightly sealed bladder obtained by fusing was not exposed with 8 Mrad of cobalt-60 as a source in a nitrogen atmosphere at 20 °C.

### Example 6

An insole 1f was obtained by the same manner as of Example 5 except for introducing an aqueous propylene glycol solution (concentration of 50% by weight) in place of propylene glycol.

### Example 7

The procedure of Example 5 was repeated except for using glycerin instead of propylene glycol, to obtain an insole 1g.

### Example 8

The procedure of Example 5 was repeated except that glycerin was introduced in place of propylene glycol and the use of the cushioning layer (6) of suede type synthetic leather and with a roughly same shape of the insole was omitted, to obtain an insole 1h.

### Example 9

A pair of ethylene-vinyl acetate copolymer sheets 42(43) having the vinyl acetate content of 10% by weight, the number-average molecular weight of about 50,000, and the thickness of 0.45 mm was cut in the shape of a shoe sole in combination with a protruded part 51 from the shoe sole, as shown in FIG. 4. The insole having this shape had such a function as ① for correcting a flatfoot by forming an arch on a flatfoot with a pressing force of a liquid to be generated when a load is exerted on the toe tip part and the heel part thereof in actual use, or ② for promoting health by simulating an entire arch of a normal person with a pressing force obtainable by the exercise of stepping and generated when a load is exerted on the toe tip part and the heel part thereof in actual use. After cutting the sheets in the above-mentioned shape, as shown in FIG. 4, the circumference part 4, barrier parts 48a to 48g, and inner fused parts 50a to 50c of the sheets 42(43), except for a portion of the heel side thereof, were fused in a fusing width of about 5 mm by means of high-frequency fusing with a maximum current of 1.20 A and for an irradiation time of 6 seconds, to form a tightly sealed bladder.

To the thus obtained tightly sealed bladder, 8 Mrad of cobalt-60 as a source is exposed in a nitrogen atmosphere at 20 °C. In this case, the cross-linking degree of the irradiated ethylene-vinyl acetate copolymer was 85%.

Then, about 20 ml of propylene glycol was introduced as a liquid (45) in the produced tightly sealed bladder from the unfused part thereof. Thereafter, this unfused part was high-frequency fused under the same conditions as mentioned above.

A cushioning layer (46) composed of a suede type synthetic leather having a roughly same shape as of the obtained insole 41 was attached to the sheet 42 side thereof (the surface contacting with the shoe sole) with chloroprene rubber (produced by Denki Kagaku Kogyo K.K. under product code "M-120 type"), to obtained an insole 41a.

### Example 10

The procedure of Example 9 was repeated except for introducing an aqueous propylene glycol solution (concentration of 50% by weight) in place of propylene glycol, to obtain an insole 41b.

### Example 11

An insole 41c was obtained by the same manner as of Example 9 except for introducing glycerin instead of propylene glycol.

### Example 12

The procedure of Example 9 was repeated except that glycerin was introduced in place of propylene glycol and the use of the cushioning layer (46) of suede type synthetic leather and with a roughly same shape of the insole was omitted, to obtain an insole 41d.

### Example 13

An insole 41e was obtained by the same manner as of Example 9 except that a pair of polyurethane sheets 42(43) with a thickness of 0.6 mm was used instead of the ethylene-vinyl acetate copolymer sheets 42(43) having the vinyl acetate content of 10% by weight, the number-average molecular weight of about 50,000, and the thickness of 0.45 mm, and the tightly sealed bladder obtained by fusing was not exposed with 8 Mrad of cobalt-60 as a source in a nitrogen atmosphere at 20 °C.

### Example 14

An insole 41f was obtained by the same manner as of Example 13 except for introducing an aqueous propylene glycol solution (concentration of 50% by weight) in place of propylene glycol.

### Example 15

The procedure of Example 13 was repeated except for using glycerin instead of propylene glycol, to obtain an insole 41g.

### Example 16

The procedure of Example 13 was repeated except that glycerin was introduced in place of propylene glycol and the use of the cushioning layer (46) of suede type synthetic leather and with a roughly same shape of the insole was omitted, to obtain an insole 41h.

### Control 1

A pair of ethylene-vinyl acetate copolymer sheets having the vinyl acetate content of 10% by weight, the number-average molecular weight of about 50,000, and the thickness of 0.45 mm was cut in the shape of a shoe sole which comprises a central part [provided that the shoe sole is divided into three parts, i.e. a toe tip part, a central arch part and a heel part]. After cutting the sheets in the above-mentioned shape, the parts corresponding to the circumference part and barrier part of the two sheets, except for a portion of the heel side thereof, were fused in a fusing width of about 5 mm by means of high-frequency fusing with a maximum current of 1.20 A and for an irradiation time of 6 seconds, to form a tightly sealed bladder.

To the thus obtained tightly sealed bladder, 8 Mrad of cobalt-60 as a source is exposed in a nitrogen atmosphere at 20 °C. In this case, the cross-linking degree of the irradiated ethylene-vinyl acetate copolymer was 85%.

Then, about 20 ml of propylene glycol was introduced as a liquid in the produced tightly sealed bladder from the unfused part thereof. Thereafter, this unfused part was high-frequency fused under the same conditions as mentioned above.

A cushioning layer composed of a suede type synthetic leather having a roughly same shape as of the obtained insole as attached to the sheet side thereof (the surface contacting with the shoe sole) with chloroprene rubber (produced by Denki Kagaku Kogyo K.K. under product code "M-120 type"), to obtained an insole 101-a.

### Control 2

The procedure of Control 1 was repeated except for introducing an aqueous propylene glycol solution (concentration of 50% by weight) in place of propylene glycol, to obtain an insole 101-b.

### Control 3

An insole 101-c was obtained by the same manner as of Control 1 except for introducing glycerin instead of propylene glycol.

### Control 4

The procedure of Control 1 was repeated except that glycerin was introduced in place of propylene glycol and the use of the cushioning layer of suede type synthetic leather and with a roughly same shape of the insole was omitted, to obtain an insole 101-d.

### Control 5

An insole 101-e was obtained by the same manner as of Control 1 except that a pair of polyurethane sheets with a thickness of 0.6 mm was used instead of the ethylene-vinyl acetate copolymer sheets having the vinyl acetate content of 10% by weight, the number-average molecular weight of about 50,000, and the thickness of 0.45 mm, and the tightly sealed bladder obtained by fusing was not exposed with 8 Mrad of cobalt-60 as a source in a nitrogen atmosphere at 20 °C

### Control 6

An insole 101-f was obtained by the same manner as of Control 5 except for introducing an aqueous propylene glycol solution (concentration of 50% by weight) in place of propylene glycol.

### Control 7

The procedure of Control 5 was repeated except for using glycerin instead of propylene glycol, to obtain an insole 101-g.

### Control 8

The procedure of Control 5 was repeated except that glycerin was introduced in place of propylene glycol and the use of the cushioning layer of suede type synthetic leather and with a roughly same shape of the insole was omitted, to obtain an insole 101-h.

### Control 9

A pair of ethylene-vinyl acetate copolymer sheets 102 and 103 having the vinyl acetate content of 10% by weight, the number-average molecular weight of about 50,000, and the thickness of 0.45 mm was cut in the shape of a shoe sole, as shown in FIG. 6. After cutting the sheets in the above-mentioned shape, as shown in FIG. 6, the circumference part 104, barrier parts 108a to 108d, and inner fused parts 110a to 110c of the sheets 102 and 103, except for a portion of the heel side thereof, were fused in a fusing width of about 5 mm by means of high-frequency fusing with a maximum current of 1.20 A and for an irradiation time of 6 seconds, to form a tightly sealed bladder. To the thus obtained tightly sealed bladder, 8 Mrad of cobalt-60 as a source is exposed in a nitrogen atmosphere at 20 °C. In this case, the cross-linking degree of the irradiated ethylene-vinyl acetate copolymer was 85%. Then, about 20 ml of propylene glycol was introduced as a liquid 105 in the produced tightly sealed bladder from the unfused part thereof. Thereafter, this unfused part was high-frequency fused under the same conditions as mentioned above. A cushioning layer 106 composed of a suede type synthetic leather having a roughly same shape as of the obtained insole was attached to the sheet 102 side thereof (the surface contacting with the shoe sole) with chloroprene rubber (produced by Denki Kagaku Kogyo K.K. under product code "M-120 type"), to obtained an insole 101-i.

### Control 10

The procedure of Control 9 was repeated except for introducing an aqueous propylene glycol solution (concentration of 50% by weight) in place of propylene glycol, to obtain an insole 101-j.

### Control 11

An insole 101-k was obtained by the same manner as of Control 9 except for introducing glycerin instead of propylene glycol.

### Control 12

The procedure of Control 9 was repeated except that glycerin was introduced in place of propylene glycol and the use of the cushioning layer 106 of suede type synthetic leather and with a roughly same shape of the insole was omitted, to obtain an insole 101-l.

### Control 13

An insole 101-m was obtained by the same manner as of Control 9 except that a pair of polyurethane sheets 102 and 103 with a thickness of 0.6 mm was used instead of the ethylene-vinyl acetate copolymer sheets 102 and 103 having the vinyl acetate content of 10% by weight, the number-average molecular weight of about 50,000, and the thickness of 0.45 mm, and the tightly sealed bladder obtained by fusing was not exposed with 8 Mrad of cobalt-60 as a source in a nitrogen atmosphere at 20 °C.

### Control 14

An insole 101-n was obtained by the same manner as of Control 13 except for introducing an aqueous propylene glycol solution (concentration of 50% by weight) in place of propylene glycol.

### Control 15

The procedure of Control 13 was repeated except for using glycerin instead of propylene glycol, to obtain an insole 101-o.

### Control 16

The procedure of Control 13 was repeated except that glycerin was introduced in place of propylene glycol and the use of the cushioning layer 106 of suede type synthetic leather and with a roughly same shape of the insole was omitted, to obtain an insole 101-p.

### Practical Tests

16 nurses subjected under test, who are 25-35 years old, have normal arches and are obliged to work standing for a long time, used in each of the shoes the insoles 1a to 1h and 41a to 41h to be obtained in Examples 1 to 16 as well as insoles 101-a to 101-p to be obtained in Controls 1 to 16, respectively.

As a result, with any of the insoles 1a to 1h and 41a to 41h to be obtained in Examples 1 to 16, fatigue from feet was remarkably alleviated without remaining fatigue to the next day. Conversely, with any of the conventional insoles 101-a to 101-p to be obtained in Controls 1 to 16, the nurses under test felt stricter fatigue as compared with those of this invention, and many nurses felt remaining fatigue until next day. In the present test, no details about the sample insoles were informed to the nurses in advance. Through questionnaires after the tests, the results were summarized.

The same kind of tests were carried out by 16 flat-footed patients all of who are adult men with 25 to 35 years old. As a result, the results were obtained similar to those of the above tests. Further, since the patients could use consecutively for a long period of days comparatively easily, they used sample insoles every day in order to check whether these insoles have flatfoot correcting effects with time. As a result, it was demonstrated from the medical diagnostic results that all the insoles produced in Examples have an effect of correcting flatfoot, although the period of starting to manifest this effect was different depending on the tested individuals. On the other hand, it was also demonstrated from the medical diagnostic results that the insoles produced in Controls had no effects of correcting flatfoot and of improving flatfoot even after the long use, although they contribute to recover fatigue to some extent by their massaging effects.

## Claims

1. An insole which comprises a tightly sealed bladder formed of two flexible resin sheets having the edged parts thereof fused and a liquid filled in the bladder to such an extent as that the two sheets partially come into mutual contact when a certain load is exerted thereon, characterized in that a protruded part is formed from said shoe sole-shaped tightly sealed bladder.

2. An insole according to claim 1, wherein said protruded part is formed for the purpose of correcting a flatfoot by the pressing force of the filled liquid to be generated when a load is exerted on a toe tip part, a heel part or a surrounding part of the arch and to produce an arch on the flatfoot.

3. An insole according to claim 1, wherein said protruded part is formed for the purpose of stimulating an whole area of an arch with the pressing force to be generated when a load is exerted on a toe tip part, a heel part or a surrounding part of the arch and to be equivalent to that produced by the exercise of stepping, and consequently can promote health.

4. An insole according to any of claims 1 to 3, wherein said liquid is at least one member selected from the group consisting of water and polyhydric alcohols.

5. An insole according to claim 4, wherein said liquid is either propylene glycol or glycerin.

6. An insole according to claim 4, wherein said liquid is an aqueous polyhydric alcohol solution.

7. An insole according to claim 6, wherein the water content of said aqueous polyhydric alcohol solution is in the range of 30 to 70% by weight.

8. An insole according to any of claims 1 to 7, wherein the thickness of the flexible resin sheet is in the range of 0.3 to 1.0 mm.

9. An insole according to any of claims 1 to 8, wherein the flexible resin material of the flexible resin sheet is at least one member selected from the group consisting of polyurethane, polyvinyl chloride, and an irradiated ethylene-vinyl acetate copolymer.

10. An insole according to claim 9, wherein the vinyl acetate content of said irradiated ethylene-vinyl acetate copolymer is in the range of 5 to 30% by weight.

11. An insole according to claim 9 or 10, wherein the irradiation is carrier out with electron rays or gamma rays.

12. An insole according to any of claims 9 to 11, wherein the irradiation dose is in the range of 4 to 10 Mrad.

13. An insole according to any of claims 9 to 12, wherein the degree of cross-linking of the irradiated ethylene-vinyl acetate copolymer is in the range of 70 to 95%.

14. An insole according to any of claims 1 to 13, wherein a cushioning layer is provided on the surface of the tightly sealed bladder which contacts with a foot sole.

15. An insole according to claim 14, wherein said cushioning layer is formed of at least one member selected from the group consisting of a woven fabric, a non-woven fabric, a raised fabric, a knitted fabric, a vinyl leather, a natural leather and a synthetic leather.

16. An insole according to any of claims 1 to 15, wherein an insole-fixing material is provided on the surface of the tightly sealed bladder which contacts with the surface of the shoe sole.

17. An insole according to claim 16, wherein a peelable and pressure-sensitive adhesive tape is used as said insole-fixing material.

18. An insole according to claim 16 or 17, wherein said insole-fixing material comprises a releasable pressure-sensitive adhesive layer, a substrate, and a releasable pressure-sensitive adhesive layer.

19. An insole according to claim 16 or 17, wherein said insole-fixing material comprises a releasable pressure-sensitive adhesive layer, a substrate, and an adhesive layer.

20. An insole according to any of claims 1 to 15, wherein said tightly sealed bladder comprises a central part and a heel part, provided in that a shoe sole is divided into a toe tip part, central part and a heel part.

21. An insole according to claim 20, wherein an insole-fixing material is provided on the surface of the tightly sealed bladder which contacts with the surface of the shoe sole.

22. An insole according to claim 21, wherein a peelable and pressure-sensitive adhesive tape is used as the insole-fixing material.

23. An insole according to claim 21 or 22, wherein said insole-fixing material comprises a releasable pressure-sensitive adhesive layer, a substrate, and a releasable pressure-sensitive adhesive layer.

24. An insole according to claim 21 or 22, wherein said insole-fixing material comprises a releasable pressure-sensitive adhesive layer, a substrate, and an adhesive layer.
